# EUROPÄISCHE PATENTANMELDUNG

(11) **EP 0 623 610 A1**
(43) Veröffentlichungstag der Anmeldung: **09.11.1994**
(21) Anmeldenummer: 94106318.2
(22) Anmeldetag: 22.04.1994
(51) Int. Cl.: C07D 401/10, A61K 31/41

(54) **Substituierte Pyridine und 2-Oxo-1,2-dihydropyridine als Angiotensin II Antagonisten**

(30) Priorität: 06.05.1993 DE 4314963
(71) Anmelder: BAYER AG, 51368 Leverkusen (DE)
(72) Erfinder: Fey, Peter, Dr., D-42111 Wuppertal (DE); Dressel, Jürgen, Dr., D-42477 Radevormwald (DE); Hanko, Rudolf, Dr., D-45134 Essen (DE); Hübsch, Walter, Dr., D-42113 Wuppertal (DE); Krämer, Thomas, Dr., D-42111 Wuppertal (DE); Müller, Ulrich, Dr., D-42111 Wuppertal (DE); Müller-Gliemann, Matthias, Dr., D-42697 Solingen (DE); Beuck, Martin, Dr., D-40699 Erkrath (DE); Bischoff, Hilmar, Dr., D-42113 Wuppertal (DE); Wohfeil, Stefan, Dr., D-40724 Hilden (DE); Denzer, Dirk, Dr., D-42115 Wuppertal (DE); Kazda, Stanislav, Dr., D-42115 Wuppertal (DE); Stasch, Johannes-Peter, Dr., D-42651 Solingen (DE); Knorr, Andreas, Dr., D-40699 Erkrath (DE); Zaiss, Siegfried, Dr., D-42113 Wuppertal (DE)

(57) **Zusammenfassung**

Substituierte Pyridine und 2-Oxo-1,2-dihydropyridine werden hergestellt durch Umsetzung von entsprechend substituierten Halogenbenzolen mit Tetrazolylboronsäuren. Die erfindungsgemäßen substituierten Pyridine und 2-oxo-1,2-dihydropyridine der allgemeinen Formel (I) 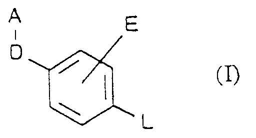
in welcher
- A: für einen Rest der Formel 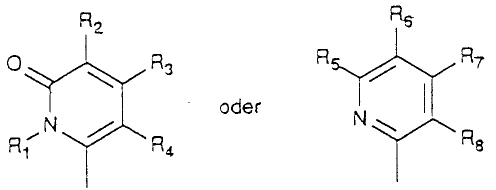
steht,
- L: für einen Rest der Formel 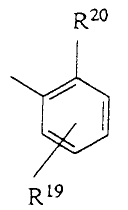
steht,
können als Wirkstoffe in Arzneimitteln, insbesondere zur Behandlung der arteriellen Hypertonie sowie Atheerosklerose eingesetzt werden.

## Beschreibung

Die Erfindung betrifft substituierte Pyridine und 2-Oxo-1,2-dihydropyridine, Verfahren zu ihrer Herstellung sowie ihre Verwendung in Arzneimitteln, insbesondere als blutdrucksenkende und anti-atherosklerotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigernden Oktapeptid Angiotensin II abgebaut wird. Die verschiedenen Effekte des Angiotensin II, wie beispielsweise Vasokonstriktion, Na⁺-Retention in der Niere, Aldosteronfreisetzung in der Nebenniere und Tonuserhöhung des sympathischen Nervensystems wirken synergistisch im Sinne einer Blutdruckerhöhung.

Darüber hinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Außerdem sind aus den Publikationen EP 407 342, 424 317, 435 827 und 419 048 Biphenyl-substituierte Pyrimidone bekannt.

Die vorliegende Erfindung betrifft substituierte Pyridine und 2-Oxo-1,2-dihydropyridine der allgemeinen Formel (I) 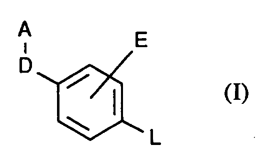
in welcher
- A: für einen Rest der Formel 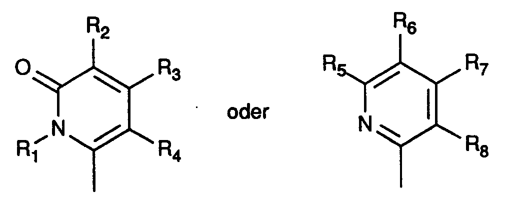 steht, worin
- R¹: geradkettiges oder verzweigtes Alkyl mit bis zu 10 Kohlenstoffatomen bedeutet, das gegebenenfalls durch Cycloalkyl mit 3 bis 6 Kohlenstoffatomen, Hydroxy oder durch geradkettiges oder verzweigtes Alkoxy oder Alkylthio mit jeweils bis zu 6 Kohlenstoffatomen substituiert ist, oder
Cycloalkyl mit 3 bis 6 Kohlenstoffatomen bedeutet,
- R², R⁵ und R⁶: gleich oder verschieden sind und
Wasserstoff, Hydroxy, Nitro, Cyano, Formyl oder Halogen bedeuten, oder
geradkettiges oder verzweigtes Alkyl, Alkenyl, Alkinyl, Alkoxy oder Alkylthio mit jeweils bis zu 8 Kohlenstoffatomen bedeuten, die gegebenenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5-bis 7-gliedrigen, gesättigten oder ungesättigten Heterocyclus mit bis zu 3 Heteroatomen aus der Reihe S, H oder O substituiert sind, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können, oder
geradkettiges oder verzweigtes Acyl oder Alkoxycarbonyl mit jeweils bis zu 8 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 6 Kohlenstoffatomen substituiert ist, das seinerseits durch Cyano, Halogen, Carboxy, Phenoxycarbonyl, Hydroxy oder durch geradkettiges oder verzweigtes Alkoxy oder Alkoxycarbonyl mit jeweils bis zu 6 Kohlenstoffatomen substituiert sein kann, oder
eine Gruppe der Formel 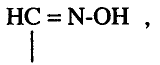 -NR⁹R¹⁰, -CO-NR¹¹R¹², -CH₂-OR¹³ oder -S(O)ₐ-R¹⁴ bedeuten
worin
- R⁹, R¹⁰, R¹¹ und R¹²: gleich oder verschieden sind und Wasserstoff, Cycloalkyl mit 3 bis 8 Kohlenstoffatomen oder geradkettiges oder verzweigtes Alkyl mit bis zu 8 Kohlenstoffatomen bedeuten, das gegebenenfalls durch Phenyl substituiert ist,
oder
- R⁹ und R¹⁰: gemeinsam mit dem Stickstoffatom einen 5- bis 7-gliedrigen, gesättigten Heterocyclus mit bis zu 2 weiteren Heteroatomen aus der Reihe S, N oder O bilden,
- R¹³: geradkettiges oder verzweigtes Acyl mit bis zu 6 Kohlenstoffatomen oder Benzoyl bedeutet,
- R¹⁴: geradkettiges oder verzweigtes Alkyl mit bis zu 8 Kohlenstoffatomen bedeutet,
- a: eine Zahl 1 oder 2 bedeutet,
- R³ und R⁷: gleich oder verschieden sind und Wasserstoff, Hydroxy, Carboxy, geradkettiges oder verzweigtes Alkoxy oder Alkoxycarbonyl mit jeweils bis zu 8 Kohlenstoffatomen bedeuten, oder
eine Gruppe der Formel -NR¹⁵R¹⁶ bedeuten,
worin
- R¹⁵ und R¹⁶: die oben angegebene Bedeutung von R⁹ und R¹⁰ haben und mit diesen gleich oder verschieden sind,
oder Aryl mit 6 bis 10 Kohlenstoffatomen bedeuten, das gegebenenfalls bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert ist, oder
geradkettiges oder verzweigtes Alkyl oder Alkenyl mit jeweils bis zu 8 Kohlenstoffatomen bedeuten, die gegebenenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können,
- R⁴: Wasserstoff, Nitro, Carboxy oder geradkettiges oder verzweigtes Alkoxycarbonyl mit bis zu 8 Kohlenstoffatomen bedeutet, oder eine Gruppe der Formel -NR¹⁷R¹⁸ bedeutet,
worin
- R¹⁷ und R¹⁸: die oben angegebene Bedeutung von R⁹ und R¹⁰ haben und mit diesen gleich oder verschieden sind,
oder
geradkettiges oder verzweigtes Alkyl oder Alkenyl mit jeweils bis zu 8 Kohlenstoffatomen bedeutet, die gegebenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können,
- R⁸: die oben angegebene Bedeutung von R¹ und R⁴ hat und mit diesen gleich oder verschieden ist,
- D: für die 〉C=O -Gruppe steht, oder
für eine Gruppe der Formel 〉CH-T steht,
worin
- T: Wasserstoff oder geradkettiges oder verzweigtes Alkyl mit bis zu 8 Kohlenstoffatomen bedeutet,
- E: für Wasserstoff, Halogen, Cyano, Nitro, Trifluormethyl, Hydroxy, Trifluormethoxy, Amido oder für geradkettiges oder verzweigtes Alkyl, Alkoxy oder Alkoxycarbonyl mit jeweils bis zu 6 Kohlenstoffatomen steht,
- L: für einen Rest der Formel 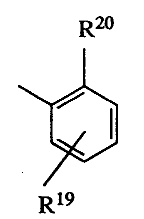 steht,
worin
- R¹⁹: die oben angegebene Bedeutung von E hat und mit dieser gleich oder verschieden ist,
und
- R²⁰: eine Gruppe der Formel -CO-R²¹, -SO₂R²², -CO-NR²³R²⁴, -NH-SO₂R²⁵ oder -SO₂-NR²⁶R²⁷ bedeutet,
worin
- R²¹: Hydroxy oder geradkettiges oder verzweigtes Alkoxy mit bis zu 6 Kohlenstoffatomen bedeutet,
- R²²: Hydroxy, Trifluormethyl, geradkettiges oder verzweigtes Alkoxy oder Alkyl mit jeweils bis zu 6 Kohlenstoffatomen, Phenyl oder Benzyl bedeutet, das gegebenenfalls bis zu 2-fach gleich oder verschieden durch Halogen, Trifluormethyl oder geradkettiges oder verzweigtes Alkyl mit bis zu 4 Kohlenstoffatomen substituiert sein kann,
- R²³ und R²⁴: gleich oder verschieden sind und die oben angegebene Bedeutung von R⁹ und R¹⁰ haben,
oder
- R²³: Wasserstoff bedeutet
und
- R²⁴: die Gruppe -SO₂R²² bedeutet,
worin
- R²²: die oben angegebene Bedeutung hat,
- R²⁵: die oben angegebene Bedeutung von R²² hat und mit dieser gleich oder verschieden ist,
- R²⁶ und R²⁷: die oben angegebene Bedeutung von R⁹ und R¹⁰ haben und mit diesen gleich oder verschieden sind,
oder
- R²⁶: geradkettiges oder verzweigtes Alkyl mit bis zu 4 Kohlenstoffatomen oder Wasserstoff bedeutet
und
- R²⁷: die oben angegebene Bedeutung von R²² hat und mit dieser gleich oder verschieden ist
oder
- R²⁰: einen Rest der Formel 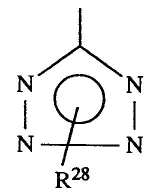 bedeutet,
worin
- R²⁸: Wasserstoff oder geradkettiges oder verzweigtes Alkyl mit bis zu 8 Kohlenstoffatomen bedeutet, das gegebenenfalls durch geradkettiges oder verzweigtes Acyl mit bis zu 6 Kohlenstoffatomen substituiert ist oder die Triphenylmethylgruppe bedeutet,
und deren Salze.

Die erfindungsgemäßen substituierten Pyridine und 2-Oxo-1,2-dihydropyridine können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe oder einen Tetrazolylrest besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Heterocyclus steht im allgemeinen für einen 5- bis 7-gliedrigen, bevorzugt 5-bis 6-gliedrigen, gesättigten oder ungesättigten Ring, der als Heteroatome bis zu 2 Sauerstoff-, Schwefel- und/oder Stickstoffatome enthalten kann. Bevorzugt sind 5-und 6-gliedrige Ringe mit einem Sauerstoff-, Schwefel- und/oder bis zu 2 Stickstoffatomen. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl, Piperazinyl oder Tetrazolyl.

Ein 5- bis 6-gliedriger, gesättigter Heterocyclus, der außerdem als Heteroatome bis zu 2 Sauerstoff-, Schwefel- und/oder Stickstoffatome enthalten kann, steht im allgemeinen für Azetidinyl, Piperidyl, Morpholinyl, Piperazinyl oder Pyrrolidyl. Bevorzugt ist Morpholinyl.

Bevorzugt sind Verbindungen der allgemeinen Formel (I),
in welcher
- A: für einen Rest der Formel 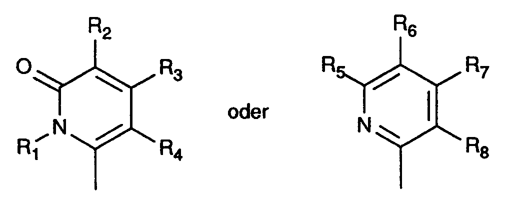 steht,
worin
- R¹: Wasserstoff, geradkettiges oder verzweigtes Alkyl mit jeweils bis zu 8 Kohlenstoffatomen bedeutet, das gegebenenfalls durch Cyclopropyl, Cyclopentyl, Cyclohexyl, Hydroxy oder durch geradkettiges oder verzweigtes Alkoxy oder Alkylthio mit jeweils bis zu 4 Kohlenstoffatomen substituiert ist, oder Cyclopropyl, Cyclopentyl oder Cyclohexyl bedeutet,
- R², R⁵ und R⁶: gleich oder verschieden sind und
Wasserstoff, Hydroxy, Nitro, Cyano, Formyl, Fluor, Chlor, Brom oder Iod bedeuten, oder
geradkettiges oder verzweigtes Alkyl, Alkenyl, Alkinyl, Alkoxy oder Alkylthio mit jeweils bis zu 6 Kohlenstoffatomen bedeuten, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können, oder
geradkettiges oder verzweigtes Acyl oder Alkoxycarbonyl mit jeweils bis zu 6 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 5 Kohlenstoffatomen substituiert ist, das seinerseits durch Cyano, Fluor, Chlor, Brom, Carboxy, Hydroxy oder durch geradkettiges oder verzweigtes Alkoxy oder Alkoxycarbonyl mit jeweils bis zu 4 Kohlenstoffatomen substituiert sein kann, oder
eine Gruppe der Formel 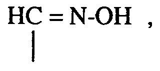 -NR⁹R¹⁰, -CO-NR¹¹R¹², -CH₂-OR¹³ oder -S(O)ₐ-R¹⁴ bedeuten
worin
- R⁹, R¹⁰, R¹¹ und R¹²: gleich oder verschieden sind und Wasserstoff, Cyclopropyl, Cyclopentyl, Cyclohexyl oder geradkettiges oder verzweigtes Alkyl mit bis zu 6 Kohlenstoffatomen bedeuten, das gegebenenfalls durch Phenyl substituiert ist,
oder
- R⁹ und R¹⁰: gemeinsam mit dem Stickstoffatom einen Morpholinring bilden,
- R¹³: geradkettiges oder verzweigtes Acyl mit bis zu 6 Kohlenstoffatomen oder Benzoyl bedeutet,
- R¹⁴: geradkettiges oder verzweigtes Alkyl mit bis zu 6 Kohlenstoffatomen bedeutet,
- a: eine Zahl 1 oder 2 bedeutet,
- R³ und R⁷: gleich oder verschieden sind und Wasserstoff, Hydroxy, Carboxy, geradkettiges oder verzweigtes Alkoxy oder Alkoxycarbonyl mit jeweils bis zu 6 Kohlenstoffatomen bedeuten, oder
eine Gruppe der Formel -NR¹⁵R¹⁶ bedeuten,
worin
- R¹⁵ und R¹⁶: die oben angegebene Bedeutung von R⁹ und R¹⁰ haben und mit diesen gleich oder verschieden sind,
oder
Phenyl bedeuten, das gegebenenfalls durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein kann, oder
geradkettiges oder verzweigtes Alkyl oder Alkenyl mit jeweils bis zu 6 Kohlenstoffatomen bedeuten, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können,
- R⁴: Wasserstoff, Nitro, Carboxy oder geradkettiges oder verzweigtes Alkoxycarbonyl mit bis zu 6 Kohlenstoffatomen bedeutet, oder eine Gruppe der Formel -NR¹⁷R¹⁸ bedeutet,
worin
- R¹⁷ und R¹⁸: die oben angegebene Bedeutung von R⁹ und R¹⁰ haben und mit diesen gleich oder verschieden sind,
oder
geradkettiges oder verzweigtes Alkyl oder Alkenyl mit jeweils bis zu 6 Kohlenstoffatomen bedeutet, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können,
- R⁸: die oben angegebene Bedeutung von R¹ und R⁴ hat und mit diesen gleich oder verschieden ist,
- D: für die 〉C=O -Gruppe steht, oder
für eine Gruppe der Formel 〉CH-T steht,
worin
- T: Wasserstoff oder geradkettiges oder verzweigtes Alkyl mit bis zu 7 Kohlenstoffatomen bedeutet,
- E: für Wasserstoff, Fluor, Chlor, Brom, Cyano, Nitro, Methoxy, Amido, Trifluormethyl, Trifluormethoxy oder für geradkettiges oder verzweigtes Alkyl oder Alkoxycarbonyl mit jeweils bis zu 4 Kohlenstoffatomen steht,
- L: für einen Rest der Formel 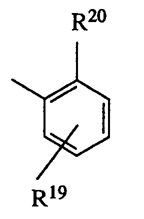 steht,
worin
- R¹⁹: die oben angegebene Bedeutung von D hat und mit dieser gleich oder verschieden ist
und
- R²⁰: eine Gruppe der Formel -CO-R²¹, -SO₂R²², -CO-NR²³R²⁴, -NH-SO₂R²⁵ oder -SO₂-NR²⁶R²⁷ bedeutet,
worin
- R²¹: Hydroxy oder geradkettiges oder verzweigtes Alkoxy mit bis zu 4 Kohlenstoffatomen bedeutet,
- R²²: geradkettiges oder verzweigtes Alkyl mit bis zu 4 Kohlenstoffatomen, Benzyl, Trifluormethyl oder p-Tolyl bedeutet,
- R²³ und R²⁴: gleich oder verschieden sind und die oben angegebene Bedeutung von R⁹ und R¹⁰ haben oder
- R²³: Wasserstoff bedeutet und
- R²⁴: die Gruppe -SO₂R²² bedeutet,
worin
- R²²: die oben angegebene Bedeutung hat,
- R²⁵: die oben angegebene Bedeutung von R²² hat und mit dieser gleich oder verschieden ist,
- R²⁶ und R²⁷: die oben angegebene Bedeutung von R⁹ und R¹⁰ haben und mit diesen gleich oder verschieden sind,
oder
- R²⁶: geradkettiges oder verzweigtes Alkyl mit bis zu 4 Kohlenstoffatomen oder Wasserstoff bedeutet
und
- R²⁷: die oben angegebene Bedeutung von R²² hat und mit dieser gleich oder verschieden ist,
oder
- R²⁰: einen Rest der Formel 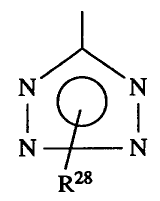 bedeutet,
worin
- R²⁸: Wasserstoff oder geradkettiges oder verzweigtes Alkyl mit bis zu 6 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,
und deren Salze.

Besonders bevorzugt sind Verbindungen der allgemeinen Formel (I),
in welcher
- A: für einen Rest der Formel 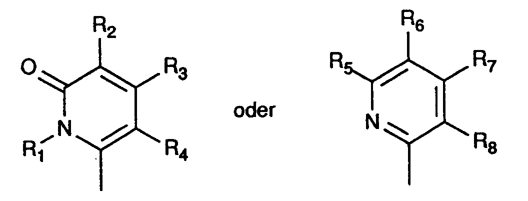 steht,
worin
- R¹: Wasserstoff oder geradkettiges oder verzweigtes Alkyl mit bis zu 6 Kohlenstoffatomen bedeutet, das gegebenenfalls durch Hydroxy, geradkettiges oder verzweigtes Alkoxy mit jeweils bis zu 3 Kohlenstoffatomen substituiert ist oder
Cyclopropyl, Cyclopentyl oder Cyclohexyl bedeutet,
- R² und R⁶: gleich oder verschieden sind und Wasserstoff, Cyano, Formyl, Fluor, Chlor, Brom oder Iod bedeuten, oder
für geradkettiges oder verzweigtes Alkyl oder Alkoxy mit jeweils bis zu 4 Kohlenstoffatomen stehen, oder
geradkettiges oder verzweigtes Alkoxycarbonyl mit jeweils bis zu 4 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 4 Kohlenstoffatomen substituiert ist, oder -NR⁹R¹⁰, -CO-NR¹¹R¹², -CH₂-OR¹³ bedeuten
worin
- R⁹, R¹⁰, R¹¹ und R¹²: gleich oder verschieden sind und Wasserstoff, Cyclopropyl, Cyclopentyl oder geradkettiges oder verzweigtes Alkyl mit bis zu 4 Kohlenstoffatomen bedeuten, das gegebenenfalls durch Phenyl substituiert ist,
- R¹³: geradkettiges oder verzweigtes Acyl mit bis zu 4 Kohlenstoffatomen oder Benzoyl bedeutet,
- R⁵: Hydroxy oder geradkettiges oder verzweigtes Alkyl oder Alkoxy mit jeweils bis zu 6 Kohlenstoffatomen bedeutet,
- R³ und R⁷: gleich oder verschieden sind und Wasserstoff, Hydroxy, Carboxy, geradkettiges oder verzweigtes Alkoxy oder Alkoxycarbonyl mit jeweils bis zu 4 Kohlenstoffatomen bedeuten, oder
eine Gruppe der Formel -NR¹⁵R¹⁶ bedeuten,
worin
- R¹⁵ und R¹⁶: die oben angegebene Bedeutung von R⁹ und R¹⁰ haben und mit diesen gleich oder verschieden sind,
oder
Phenyl, Vinyl oder geradkettiges oder verzweigtes Alkyl mit bis zu 5 Kohlenstoffatomen bedeuten,
- R⁴: Wasserstoff, Carboxy oder geradkettiges oder verzweigtes Alkoxycarbonyl mit bis zu 4 Kohlenstoffatomen bedeutet, oder
eine Gruppe der Formel -NR¹⁷R¹⁸ bedeutet,
worin
- R¹⁷ und R¹⁸: die oben angegebene Bedeutung von R⁹ und R¹⁰ haben und mit diesen gleich oder verschieden sind,
oder
Vinyl oder geradkettiges oder verzweigtes Alkyl mit bis zu 4 Kohlenstoffatomen bedeutet,
- R⁸: die oben angegebene Bedeutung von R¹ und R⁴ hat und mit diesen gleich oder verschieden ist,
- D: für die 〉C=O-Gruppe steht, oder
für eine Gruppe der Formel 〉CH-T steht,
worin
- T: Wasserstoff oder geradkettiges oder verzweigtes Alkyl mit bis zu 5 Kohlenstoffatomen bedeutet,
- E: für Wasserstoff, Fluor, Chlor, Brom, Cyano, Methoxy, Trifluormethyl oder geradkettiges oder verzweigtes Alkyl oder Alkoxycarbonyl mit jeweils bis zu 3 Kohlenstoffatomen steht,
und
- L: für einen Rest der Formel 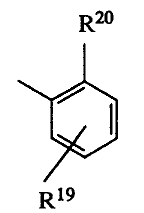 steht,
worin
- R¹⁹: Wasserstoff bedeutet,
und
- R²⁰: eine Gruppe der Formel -CO-R²¹ bedeutet,
worin
- R²¹: Hydroxy oder geradkettiges oder verzweigtes Alkoxy mit bis zu 4 Kohlenstoffatomen bedeutet,
oder
- R²⁰: den Tetrazolylrest der Formel 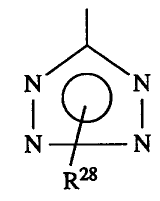 bedeutet,
worin
- R²⁸: Wasserstoff oder geradkettiges oder verzweigtes Alkyl mit bis zu 4 Kohlenstoffatomen bedeutet,
oder die Triphenylmethylgruppe bedeutet,
und deren Salze.

Ganz besonders bevorzugt sind Verbindungen der allgemeinen Formel (I),
in welcher
- A: für einen Rest der Formel 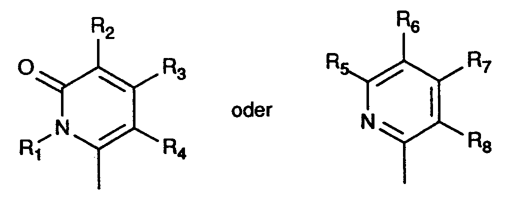 steht,
worin
- R¹: geradkettiges oder verzweigtes Alkyl mit bis zu 4 Kohlenstoffatomen bedeutet,
- R² und R⁶: gleich oder verschieden sind und Wasserstoff, Carboxy oder geradkettiges oder verzweigtes Alkoxycarbonyl mit bis zu 4 Kohlenstoffatomen bedeuten,
- R⁵: Hydroxy oder geradkettiges oder verzweigtes Alkyl oder Alkoxy mit bis zu 4 Kohlenstoffatomen bedeutet,
- R³ und R⁷: gleich oder verschieden sind und Wasserstoff, Hydroxy, Carboxy oder geradkettiges oder verzweigtes Alkoxy oder Alkoxycarbonyl mit jeweils bis zu 4 Kohlenstoffatomen bedeuten,
- R⁴: Wasserstoff, Carboxy oder geradkettiges oder verzweigtes Alkoxycarbonyl mit bis zu 4 Kohlenstoffatomen bedeutet,
- R⁸: die oben angegebene Bedeutung von R¹ und R⁴ hat und mit diesen gleich oder verschieden ist,
- D: für die 〉C=O-Gruppe steht, oder
für eine Gruppe der Formel 〉CH-T steht,
worin
- T: Wasserstoff oder geradkettiges oder verzweigtes Alkyl mit bis zu 4 Kohlenstoffatomen bedeutet,
- E: für Wasserstoff, Fluor, Chlor, Trifluormethyl oder Cyano steht,
und
- L: für einen Rest der Formel 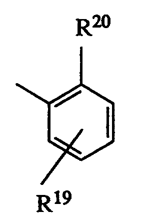 steht,
worin
- R¹⁹: Wasserstoff bedeutet,
und
- R²⁰: eine Gruppe der Formel -CO-R²¹, bedeutet,
worin
- R²¹: Hydroxy oder geradkettiges oder verzweigtes Alkoxy mit bis zu 4 Kohlenstoffatomen bedeutet,
oder
- R²⁰: den Tetrazolylrest der Formel 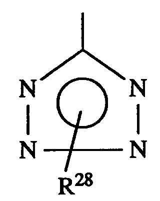 bedeutet,
worin
- R²⁸: Wasserstoff oder die Triphenylmethylgruppe bedeutet,
und deren Salze.

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (II) 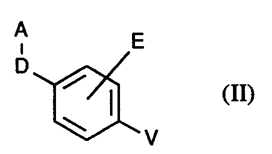
in welcher
- A, D und E: die oben angegebene Bedeutung haben
und
- V: für eine typische Abgangsgruppe, wie beispielsweise Brom, Jod, Methan-, Toluol-, Fluor- oder Trifluormethansulfonyloxy, vorzugsweise für Brom steht,
mit Verbindungen der allgemeinen Formel (III) oder (IIIa) 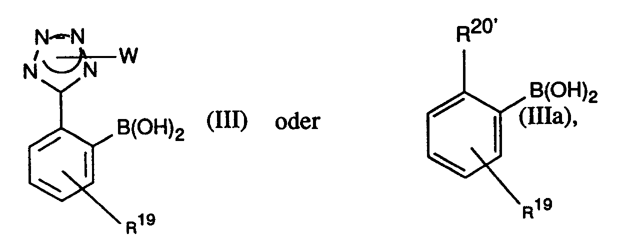
in welcher
- R¹⁹: die oben angegebene Bedeutung hat,
- W: für Wasserstoff oder für die Triphenylmethylgruppe steht,
und
- R^{20'}: die oben angegebene Bedeutung von R²⁰ hat, aber nicht für den Tetrazolylrest steht,
in inerten Lösemitteln, in Anwesenheit einer Base und metallkatalysiert umsetzt,
und anschließend im Fall daß W für die Triphenylmethylgruppe steht mit Säuren in organischen Lösemitteln und/oder Wasser nach üblichen Bedingungen abspaltet,
und gegebenenfalls im Fall der unter dem Substituenten R²⁰ aufgeführten Reste nach Verseifung der jeweiligen Ester, beispielsweise durch Amidierung oder Sulfoamidierung, nach üblichen Methoden derivatisiert,
und im Fall der Salze bevorzugt ausgehend vom freien Tetrazol (R²⁸/W = H) mit Säuren oder Basen umsetzt,
und im Fall der freien Säure R²⁰ = CO₂H und des freien Tetrazols R²⁸ = H, ausgehend von den Salzen mit Säuren umsetzt,
und gegebenenfalls auch die übrigen Substituenten nach bekannten Methoden auf jeder Verfahrensstufe variiert.

Das erfindungsgemäße Verfahren kann durch folgendes Formelschema beispielhaft erläutert werden: 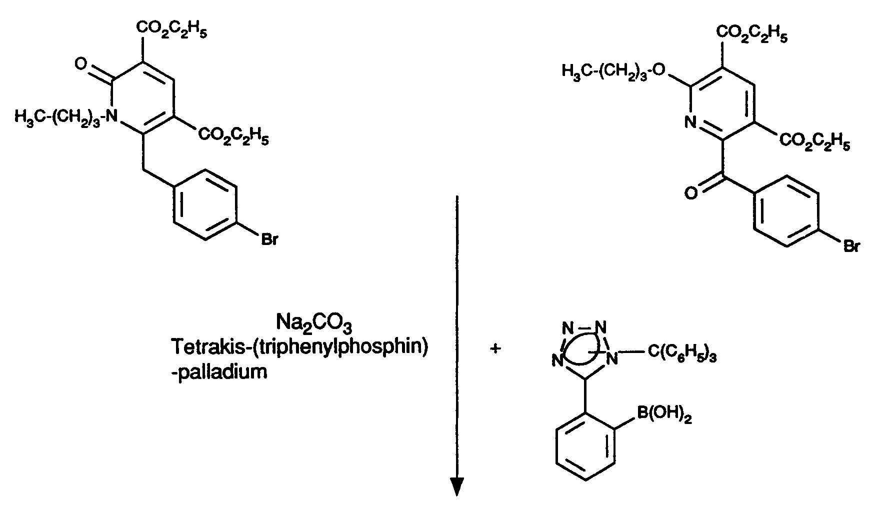 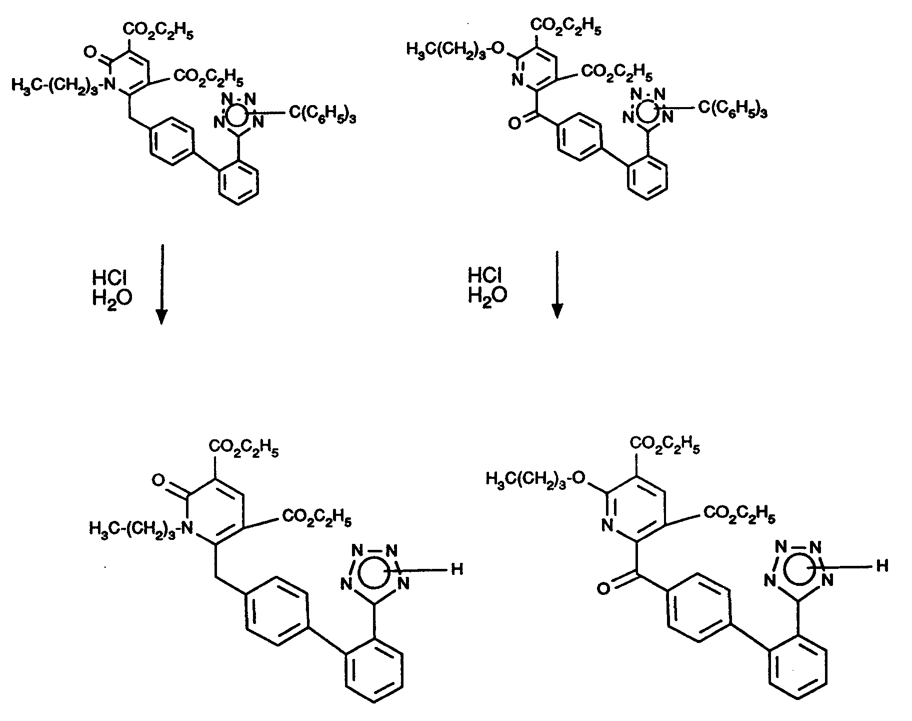
Als Lösemittel für das Verfahren eignen sich übliche organische Lösemittel, die sich unter den Reaktionsbedingungen nicht verändern. Hierzu gehören bevorzugt Wasser oder Alkohole, wie beispielsweise Methanol, Ethanol und Propanol, Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Dimethylsulfoxid, Dimethylformamid oder Dimethoxyethan, Hexamethylphosphorsäuretriamid, Acetonitril, Aceton oder Nitromethan.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat oder Caesiumcarbonat, oder Alkali- oder Erdalkalialkoholate oder -amide wie Natrium-oder Kaliummethanolat, Natrium- oder Kaliumethanolat oder Kalium-tert.butylat, Thalliumcarbonat- oder -hydroxid, oder Lithiumdiisopropylamid (LDA), oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle, wie Natrium oder deren Hydride wie Natriumhydrid einzusetzen. Bevorzugt sind Kaliumcarbonat, Natriumhydrid, Kalium-tert.-butylat, Caesiumcarbonat, Natriumcarbonat, Thalliumhydroxid oder -carbonat.

Im allgemeinen setzt man die Base in einer Menge von 0,05 mol bis 10 mol, bevorzugt von 1 mol bis 2 mol jeweils bezogen auf 1 mol der Verbindung der Formel (III) ein.

Die erfindungsgemäßen Verfahren werden im allgemeinen in einem Temperaturbereich von -100°C bis +150°C, bevorzugt von 0°C bis 100°C und Schutzgasatmosphäre durchgeführt.

Als Katalysatoren eignen sich im allgemeinen Metallkomplexe des Nickels, Palladiums oder Platins, bevorzugt Palladium(O)-Komplexe wie beispielsweise Tetrakistriphenylphosphinpalladium. Ebenso ist es möglich Phasen-Transfer-Katalysatoren, wie beispielsweise Tetra-n-butylammoniumbromid oder Kronenether einzusetzen.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Abspaltung der Triphenylmethylgruppe erfolgt mit Essigsäure oder Trifluoressigsäure und Wasser oder einem der oben aufgeführten Alkohole oder mit wäßriger Salzsäure in Anwesenheit von Aceton und Alkoholen.

Die Abspaltung erfolgt im allgemeinen in einem Temperaturbereich von 0°C bis 150°C, vorzugsweise von 20°C bis 100°C und Normaldruck.

Der Katalysator wird in einer Menge von 0,005 mol bis 0,2 mol, bevorzugt von 0,01 mol bis 0,05 mol, bezogen auf 1 mol der Verbindung der allgemeinen Formel (II) eingesetzt.

Die Alkylierung erfolgt im allgemeinen mit Alkylierungsmitteln wie beispielsweise (C₁-C₆)-Alkylhalogeniden, Sulfonsäurestern oder substituierten oder unsubstituierten (C₁-C₆)-Dialkyl- oder (C₁-C₆)-Diarylsulfonaten, vorzugsweise Methyliodid oder Dimethylsulfat.

Die Alkylierung erfolgt im allgemeinen in einem der oben aufgeführten Lösemitteln, vorzugsweise in Dimethylformamid in einem Temperaturbereich von 0°C bis +70°C, vorzugsweise von 0°C bis +30°C und Normaldruck.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemitteln und/oder Wasser oder deren Gemische, vorzugsweise mit Dioxan oder Tetrahydrofuran.

Die Amidierung und die Sulfonamidierung erfolgen im allgemeinen in einem der oben aufgeführten Lösemitteln, vorzugsweise in Tetrahydrofuran oder Dichlormethan.

Die Amidierung und die Sulfonamidierung können gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung und die Sulfonamidierung erfolgt im allgemeinen in einem Temperaturbereich von -20°C bis +80°C, vorzugsweise von -10°C bis +30°C und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 2 mol, bezogen auf 1 mol der entsprechenden Säure oder Esters, eingesetzt.

Als säurebindende Mittel für die Sulfonamidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonen-5 (DBN) oder 1,5-Diazabicyclo[3.4.0]undecen-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der allgemeinen Formel (II) sind größtenteils neu und können beispielsweise hergestellt werden, indem man
im Fall, daß A für den Rest der Formel 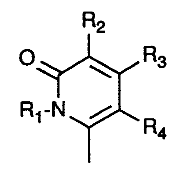
steht und D die 〉CH-T-Gruppe bedeutet,
Verbindungen der allgemeinen Formel (IV) 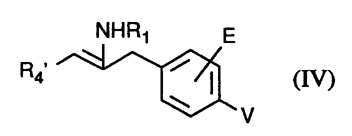
in welcher
- R¹, E und V: die oben angegebene Bedeutung haben
und
- R^{4'}: für Nitril oder für einen der oben unter R⁴ aufgeführten, chemisch sinnvollen Reste, bevorzugt für (C₁-C₄)-Alkoxycarbonyl, Nitro oder Nitril steht,
mit Verbindungen der allgemeinen Formel (V) 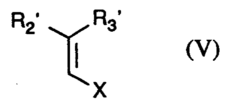
in welcher
- R^{2'} und R³: ^{'} gleich oder verschieden sind und für Nitril oder für einen der oben unter R² und R³ aufgeführten, chemisch sinnvollen Reste, bevorzugt für (C₁-C₄)-Alkoxycarbonyl oder Nitril stehen,
und
- X: für (C₁-C₄)-Alkoxy oder (C₁-C₄)-Dialkylamino steht,
umsetzt.

Die Reaktion verläuft in einem Temperaturbereich von +50°C bis +130°C, vorzugsweise von +70°C bis +110°C und Normaldruck.

Die Verbindungen der allgemeinen Formel (IV) sind teilweise bekannt und können dann beispielsweise hergestellt werden, indem man
Verbindungen der allgemeinen Formel (VI) 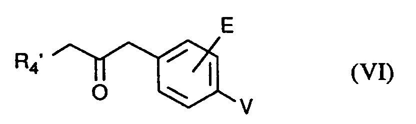
in welcher
- R^{4'}, E und V: die oben angegebene Bedeutung haben,
in einem der oben anfgeführten Lösemittel, vorzugsweise Ethanol, mit Ammoniak (R¹ = H) oder mit Aminen der allgemeinen Formel (VII)

H₂N-R¹ (VII)

in welcher
- R¹: die oben angegebene Bedeutung hat,
umsetzt.

Die Reaktion verläuft im allgemeinen in einem Temperaturbereich von - 10°C bis +100°C, vorzugsweise von 0°C bis +100°C.

Die Amine der allgemeinen Formel (VII) sind bekannt.

Die Verbindungen der allgemeinen Formel (VI) sind teilweise bekannt und können beispielsweise hergestellt werden, indem man
Verbindungen der allgemeinen Formel (VIII) 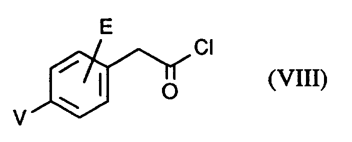
in welcher
- E und V: die oben angegebene Bedeutung haben,
mit Verbindungen der allgemeinen Formel (IX)

R^{4'}-CH₂-CO₂H (IX)

in welcher
- R^{4'}: die oben angegebene Bedeutung hat,
in einem der oben aufgeführten Lösemittel, vorzugsweise Tetrahydrofuran, umsetzt.

Die Umsetzung erfolgt in einem Temperaturbereich von 0°C bis +40°C, vorzugsweise bei +20°C.

Die Verbindungen der allgemeinen Formeln (VIII) und (IX) sind an sich bekannt.

Die Verbindungen der allgemeinen Formel (II), in welcher A für den Rest der Formel 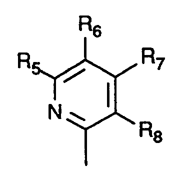
steht, sind neu und können beispielsweise hergestellt werden, indem man
Verbindungen der allgemeinen Formel (IIa) 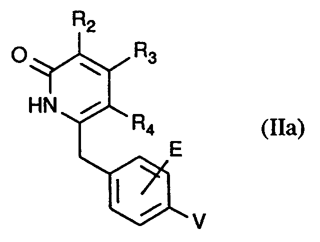
in welcher
- R², R³, R⁴, E und V: die oben angegebene Bedeutung haben,
in einem der oben aufgeführten Lösemittel, vorzugsweise Dimethylformamid, in Anwesenheit einer Base, vorzugsweise Caesiumcarbonat, zunächst mit Verbindungen der allgemeinen Formel (X)

R¹-Z (X)

in welcher
- R¹: die oben angegebene Bedeutung hat, aber vorzugsweise für (C₁-C₆)-Alkyl steht
und
- Z: für Halogen, vorzugsweise für Jod steht,
umsetzt.

Bei dieser Alkylierung entstehen außerdem die Verbindungen der allgemeinen Formel (II), in welcher D für die C=O-Gruppe steht.

Die Umsetzung erfolgt im allgemeinen in einem Temperaturbereich von 0°C bis +50°C, vorzugsweise von +10°C bis +30°C, und Normaldruck.

Die Base wird in einer Menge von 1 mol bis 10 mol, bevorzugt von 1 mol bis 3 mol bezogen auf 1 mol der Verbindungen der allgemeinen Formel (X) eingesetzt.

Die Verbindungen der allgemeinen Formel (IIa) sind neu und können wie oben beschrieben hergestellt werden.

Die Verbindungen der allgemeinen Formel (X) sind bekannt.

Die Verbindungen der allgemeinen Formel (III) sind teilweise bekannt oder im Fall W = H neu und können dann hergestellt werden, indem man Phenyltetrazol zunächst unter Schutzgasatmosphäre, in einem aprotischen Lösemittel und in Anwesenheit einer Base umsetzt, anschließend Borsäuretrimethylester zufügt und in einem letzten Schritt mit Säuren hydrolysiert.

Als Lösemittel eignen sich für das Verfahren aprotische Lösemittel wie Ether, beispielweise Tetrahydrofuran, Diethylether, Toluol, Hexan oder Benzol. Bevorzugt ist Tetrahydrofuran.

Als Basen eignen sich prim-, sec.- und tert.Butyllithium und Phenyllithium. Bevorzugt ist n-Butyllithium.

Die Base wird in einer Menge von 2 mol bis 5 mol, bevorzugt von 2 mol bis 3 mol bezogen auf 1 mol Phenyltetrazol eingesetzt.

Als Säuren eignen sich im allgemeinen Mineralsäuren, wie beispielsweise Salzsäure, C₁-C₄-Carbonsäuren, wie beispielsweise Essigsäure oder Phosphorsäuren. Bevorzugt ist Salzsäure.

Die Säure wird im allgemeinen in einer Menge von 1 mol bis 10 mol, bevorzugt von 1 mol bis 3 mol, eingesetzt.

Das Verfahren wird im allgemeinen in einem Temperaturbereich von -70°C bis +25°C, bevorzugt bei -10°C bis 0°C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (IIIa) sind teilweise neu oder können nach üblichen Methoden hergestellt werden.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt, jede Modifikation dieser Verfahren ist in gleicher Weise für die Herstellung anwendbar.

Die erfindungsgemäßen substituierten Pyridine und 2-Oxo-1,2-dihydropyridine zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimittel zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Außerdem können die Verbindungen zur Bekämpfung von Glaukom, diabetischer Retinopathie Bewegungssteigerung der intraokularen Netzhautflüssigkeit eingesetzt werden.

Sie sind auch geeignet zur Bekämpfung von Erkrankungen des zentralen Nervensystems wie beispielsweise Depression, Migräne, Schizophrenie oder Angstzuständen, von Hirnleistungsstörungen, Schlaganfall, diabetischer Nephropathie, Herzrhytmusstörungen, Prophylaxe von koronaren Herzerkrankungen oder Restenoseprophylaxe nach Angioplastien und gefäßchrirugischen Maßnahmen.

### Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogen-begaster Krebs-Henseleit-Nährlösung folgender Zusammensetzung: 119 mmol/l NaCl; 2,5 mmol/l CaCl₂ x 2 H₂O; 1,2 mmol/l KH₂PO₄; 10 mmol/l Glucose; 4,8 mmol/l KCl; 1,4 mmol/l MgSO₄ x 7 H₂O und 25 mmol/l NaHCO₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

### Agonisten und ihre Standardkonzentrationen Applikationsvolumen pro Einzelgabe = 100 µl):

| | | |
|---|---|---|
| KCl | 22,7;32,7;42,7;52,7 | mmol/l |
| Noradrenalin | 3x10⁻⁹;3x10⁻⁸;3x10⁻⁷;3x10⁻⁶ | g/ml |
| Serotonin | 10⁻⁸;10⁻⁷;10⁻⁶;10⁻⁵ | g/ml |
| B-HT 920 | 10⁻⁷;10⁻⁶;10⁻⁵ | g/ml |
| Methoxamin | 10⁻⁷;10⁻⁶;10⁻⁵ | g/ml |
| Angiotensin II | 3x10⁻⁹;10⁻⁸;3x10⁻⁸;10⁻⁷ | g/ml |

Für die Berechnung der IC₅₀ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

### Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

### Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.
Der arterielle Blutdruck dieser Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

### Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), ³H-Angiotensin II (3-5 nM), Test-Pufferlösung (50 mM Tris, pH 7,2), 5 mM MgCl₂ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu Kᵢ- bzw. IC₅₀-Werten (Kᵢ: für die verwendete Radioaktivität korrigierte IC₅₀-Werte; IC₅₀-Werte: Konzentration bei der die zu untersuchende Substanz eine 50%ige Hemmung der spezifischen Bindung des Radioliganden bewirkt).

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% CO₂ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi ³H-Thymidin zugefügt und nach weiteren 4 Stunden der Einbau dieser Substanz in die TCA-präzipitierbare DNA der Zellen bestimmt. Zur Bestimmung der IC₅₀-Werte wird die Wirkstoffkonzentration errechnet, die bei sequentieller Verdünnung des Wirkstoffes halbmaximale Hemmung der durch 10% FCS hervorgerufene Thymidininkorporation bewirkt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Ausgangsverbindungen

### Beispiel I

### 4-(4-Bromphenyl)-3-oxo-butansäureethylester

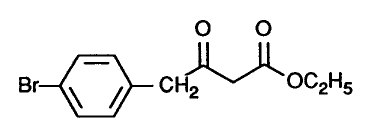

Zu einer Lösung von 125 g (0,95 mol) Malonsäuremonoethylester in 1 l Tetrahydrofuran tropft man bei 0°C 1 l (2 mol) einer 2 M Lösung von Isopropylmagnesiumchlorid in Tetrahydrofuran und rührt 1 h bei 20°C. Anschließend tropft man bei 20°C 146 g (0,63 mol) 4-Bromphenylessigsäurechlorid zu, rührt 2 h bei dieser Temperatur und stellt das Reaktionsgemisch mit 1 N Salzsäure auf pH = 3 ein. Es wird über Nacht gerührt, die Phasen werden getrennt, die wäßrige Phase wird mit Diethylether gewaschen, die vereinigten organischen Phasen werden mit Wasser und wässriger Natriumhydrogencarbonatlösung gewaschen, das Lösemittel wird abdestilliert, der Rückstand in Dichlormethan gelöst, mit Natriumsulfat getrocknet, über Kieselgel mit Dichlormethan filtriert und das Lösemittel am Rotationsverdampfer entfernt.
Rohausbeute: 167,9 g Öl, 93,5% der Theorie
R_{f} (Petrolether/Essigester 5:1, Kieselgel) = 0,43

### Beispiel II

### 3-Amino-4-(4-bromphenyl)-but-2-ensäureethylester

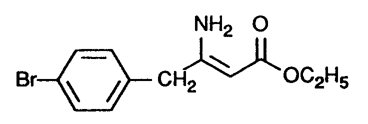

In eine Lösung von 10 g (35 mmol) der Verbindung aus Beispiel I in 200 ml Ethanol wird bei 0°C 3 h Ammoniak eingeleitet und die Lösung über Nacht auf 20°C aufgewärmt. Das Lösemittel wird im Vakuum abdestilliert, der Rückstand in Dichlormethan gelöst und über Kieselgel filtriert. Nach Entfernen des Lösemittels werden 7,8 g der Titelverbindung erhalten.
Ausbeute: 78% der Theorie
MS (EI): 283/285 (M), 284/286 (M+1), 237, 239

### Beispiel III

### 6-(4-Brombenzyl)-2-oxo-1,2-dihydropyridin-2,5-dicarbonsäurediethylester

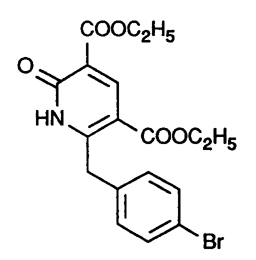

69 g (0,24 mol) der Verbindung aus Beispiel II und 52,49 g (0,24 mol) Ethoxymethylenmalonsäurediethylester werden 70 h bei 100°C gerührt. Das Reaktionsgemisch wird auf 250 g Kieselgel aufgezogen und mit Essigester / Petrolethergemischen (1:10 - 1:0) an 200 g Kieselgel eluiert. Der erhaltene braune Feststoff [R_{f} (Essigester/Petrolether 1:1, Kieselgel) = 0,28] wird mit Petrolether und Essigester/Petrolether (1:10) verrieben und über Phosphorpentoxid im Vakuum zu 18,3 g der Titelverbindung getrocknet.
Ausbeute: 18,5% der Theorie
MS 407, 409 (M⁺)

### Beispiel IV

### 6-Butoxy-2-(4-bromphenylcarbonyl)-pyridin-3,5-dicarbonsäurediethylester

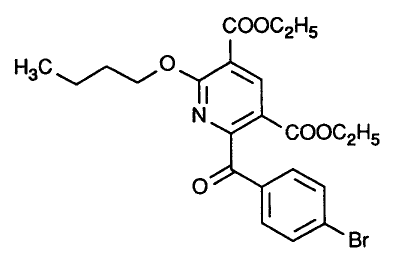

Eine Suspension von 1 g (2,45 mmol) der Verbindung aus Beispiel III, 2,8 ml (4,5 mmol) n-Butyljodid und 3,99 g (12,25 mmol) Cäsiumcarbonat in 20 ml Dimethylformamid wird über Nacht bei 20°C gerührt. Das Reaktionsgemisch wird zwischen Wasser und Essigester verteilt, die organische Phase mit Wasser und gesättigter Natriumchloridlösung gewaschen, über Natriumsulfat getrocknet und das erhaltene Rohöl (2,3 g) an 300 g Kieselgel mit Essigester/Petrolether 1:20 zu 0,13 g der Titelverbindung chromatographiert.
Ausbeute: 11,1% der Theorie
R_{f}(Kieselgel, Essigester/Petrolether 1:10) = 0,25
MS = 477, 479 (M⁺)
Weiterhin werden 0,26 g

### Beispiel V

### 6-Butoxy-2-(4-bromphenylcarbonyl)-pyridin-3,5-dicarbonsäure-5-butyl-3-ethylester

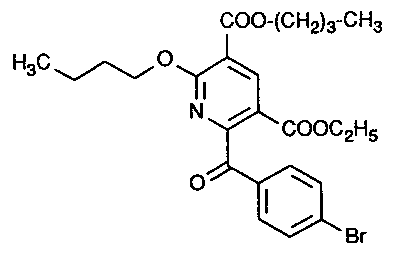

Ausbeute: 21% der Theorie
R_{f} (Kieselgel, Essigester/Petrolether 1:10)= 0,33
MS 505, 507 (M⁺)
und 93 mg

### Beispiel VI

### 6-Butoxy-2-[1-(bromphenyl-4-yl)-pentyl]-pyridin-3,5-dicarbonsäurediethylester

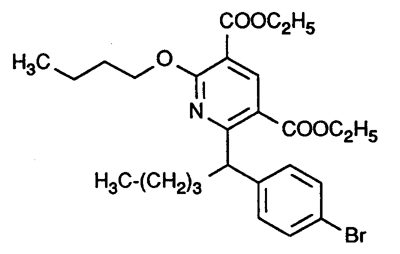

erhalten.
Ausbeute: 7,3% der Theorie
R_{f} (Kieselgel, Essigester/Petrolether 1:10) = 0,49
MS 519, 521 (M⁺)

### Beispiel VII

### 2-(Tetrazol-5-yl)phenylboronsäure

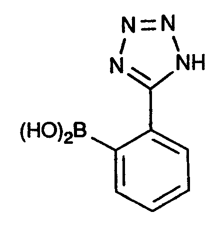

Eine Lösung von 2,9 g (20 mmol) 5-Phenyltetrazol in 50 ml THF wird bei - 5°C unter Argon mit 17,6 ml (44 mmol) einer 2,5 M Lösung von n-Butyllithium in n-Hexan versetzt. Man läßt 30 min bei -5°C bis 0°C rühren und gibt bei dieser Temperatur 10 ml (88 mmol) Borsäuretrimethylester hinzu. Dann wird das Kühlbad entfernt und die Lösung bei Raumtemperatur mit 10 ml halbkonzentrierter Salzsäure versetzt. Nach 1 h wird mit 100 ml Essigester extrahiert, die organische Phase abgetrennt und die wäßrige Phase zweimal mit je 20 ml Essigester extrahiert. Die vereinigten organischen Phasen werden über Natriumsulfat getrocknet, eingeengt und der Rückstand an Kieselgel mit Toluol / Eisessig / Methanol (38 : 0,1 : 2) gereinigt.
Ausbeute: 2,65 g (70% d.Th.)
R_{f} = 0,26 (Toluol/Methanol/Eisessig = 32:8:1)
¹³C-NMR: δ = 156,7; 137,9; 133,5; 129,8; 128,9; 127,7; 126,9 ppm.

### Beispiel VIII

### 3-Butylamino-4-(4-bromphenyl)-but-2-ensäureethylester

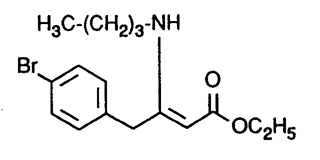

Eine Lösung von 50 g (0,175 mol) der Verbindung aus Beispiel I und 19 ml (0,195 mol) n-Butylamin in 500 ml Ethanol wird über Nacht unter Rückfluß erhitzt. Nach Einengen werden 54,3 g der Titelverbindung als Öl erhalten.
Rohausbeute: 91,2% der Theorie
R_{f} (Kieselgel, Petrolether/Essigester 10:1) = 0,52

### Beispiel IX

### 1-Butyl-2-oxo-6-(4-bromphenyl-ylmethyl)-1,2-dihydropyridin-3,5-dicarbonsäurediethylester

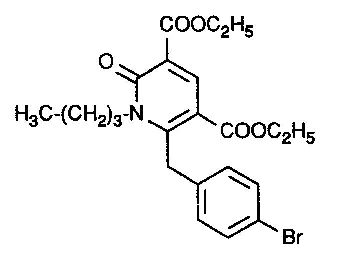

Analog Beispiel III wird die Titelverbindung ausgehend von der Verbindung aus Beispiel VIII erhalten.
R_{f} (Kieselgel, Toluol/Essigester 10:1) = 0,14

### Beispiel X

### 4-(4-Bromphenyl)-3-oxo-butansäure-methylester

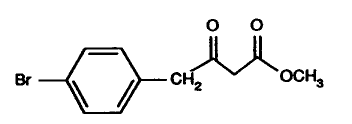

In Analogie zur Vorschrift des Beispiels I wird aus Malonsäure-monomethylester die Titelverbindung erhalten.
Ausbeute: 80 % der Theorie
R_{f} (Petrolether/Essigester 5:1, Kieselgel) = 0,38

### Beispiel XI

### 3-Butylamino-4-(4-bromphenyl)-but-2-ensäuremethylester

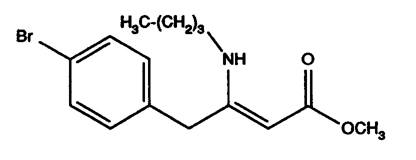

Analog zur Vorschrift des Beispiels VIII wird die Titelverbindung ausgehend von der Verbindung aus Beispiel X erhalten.
Ausbeute: 78,8 % der Theorie
R_{f} (Petrolether/Essigester 5:1, Kieselgel) = 0,63

### Beispiel XII

### 1-Butyl-2-oxo-6-(4-bromphenyl-ylmethyl)-1,2-dihydro-pyridin-3,5-dicarbonsäure-3-ethyl-5-methylester

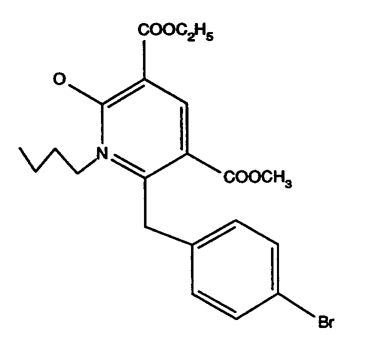

Analog zur Vorschrift des Beispiels IX wird die Titelverbindung ausgehend von der Verbindung aus Beispiel XI erhalten.
Ausbeute: 65 % der Theorie
R_{f} (Petrolether/Essigester 2:1, Kieselgel) = 0,44

### Herstellungsbeispiele

### Beispiel 1

### 6-Butoxy-2-[2'-(2-triphenylmethyl-tetrazol-5-yl)biphenyl-4-carbonyl]-pyridin-3,5-dicarbonsäurediethylester

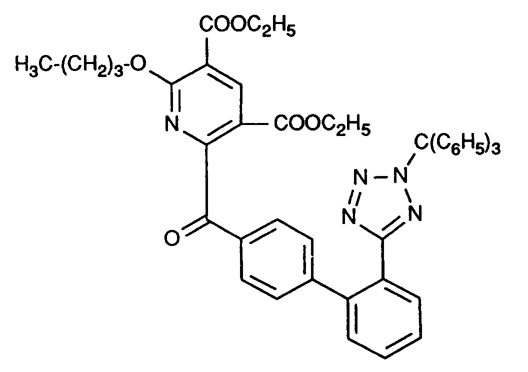

Eine Suspension aus 70,9 mg (0,15 mmol) der Verbindung aus Beispiel IV, 79,3 mg (0,18 mmol) 3-(2'-Triphenylmethyl-2'H-tetrazol-5'-yl)phenylboronsäure, 20 mg (0,18 mmol) Natriumcarbonat, 10 mg (0.008 mmol) Tetrakis-(triphenylphosphin)palladium, 0,5 ml Wasser, 0,5 ml Methanol und 3 ml Toluol wird unter Argon 4 h bei 90°C gerührt. Das Reaktionsgemisch wird zwischen Wasser und Essigester verteilt, die wäßrige Phase dreimal mit Essigester und die vereinigten organischen Phasen mit Wasser und Natriumchloridlösung gewaschen und über Natriumsulfat getrocknet. Nach Entfernen des Lösemittels wird das erhaltene Öl an 70 g Kieselgel mit Essigester /Petrolether (1:5) zu 36,9 mg der Titelverbindung chromatographiert.
Ausbeute: 30,2% der Theorie
MS (SIMS): 892 (M+Ag), 808 (M+Na)

### Beispiel 2

### 1-Butyl-2-oxo-6-[2'-(2-triphenylmethyl-tetrazolyl-5-yl)-biphenyl-4-yl-methyl]-1,2-dihydropyridin-3,5-dicarbonsäurediethylester

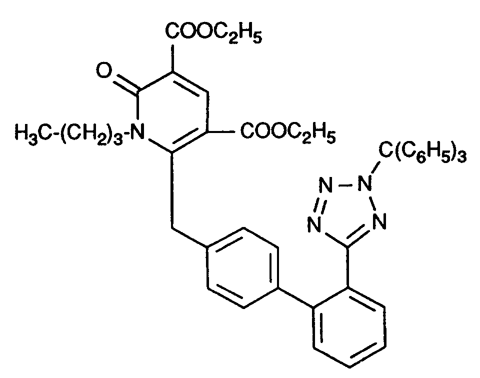

Analog Beispiel 1 wird die Titelverbindung ausgehend von der Verbindung des Beispiels IX erhalten.
R_{f} (Kieselgel, Essigester/Petrolether 1:2) = 0,38

### Beispiel 3

### 6-Butoxy-2-[2'-(1H-tetrazol-5-yl)-biphenyl-4-carbonyl]pyridin-3,5-dicarbonsäurediethylester

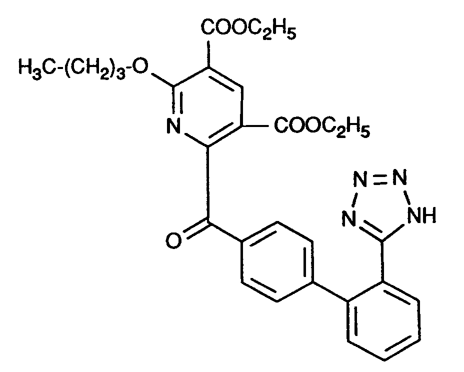

Eine Lösung von 30 mg (0,04 mmol) der Verbindung aus Beispiel 1 in 2 ml Methanol wird mit einem Tropfen konz. Salzsäure versetzt und 3 h bei 20°C gerührt. Das Reaktionsgemisch wird mit Wasser verdünnt, dreimal mit Essigester gewaschen, die vereinigten organischen Phasen werden mit Wasser und Natriumchloridlösung gewaschen, über Natriumsulfat getrocknet, eingeengt und an 25 g Kieselgel mit Dichlormethan / Methanol 10:1 zu 15,9 mg der Titelverbindung chromatographiert.
Ausbeute: 84,6% der Theorie
MS: 544 (M+1)

### Beispiel 4

### 6-Butoxy-2-[2'-(1H-tetrazol-5-yl)-biphenyl-4-carbonyl]-pyridin-3,5-dicarbonsäure-5-butyl-3-ethylester

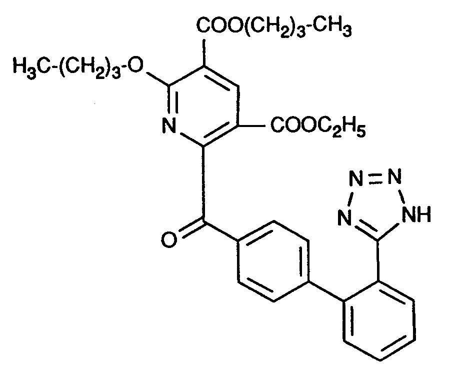

Analog Beispiel 1 und Beispiel 3 wird aus der Verbindung des Beispiels V die Titelverbindung erhalten.
MS (FAB): 572 (M+1)

### Beispiel 5

### 6-Butoxy-2-[2'-(1H-tetrazol-5-yl)-biphenyl-4-carbonyl]-pyridin-3,5-dicarbonsäure-5-butyl-3-ethylester-kaliumsalz

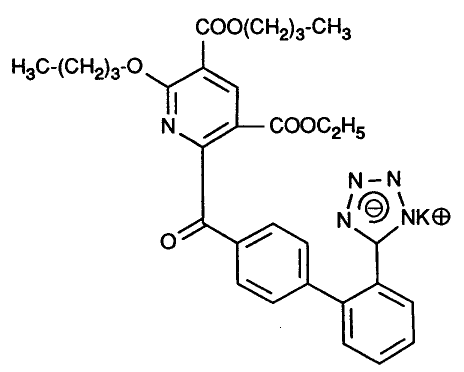

Eine Lösung von 22,7 mg (0,041 mmol) der Verbindung aus Beispiel 4 in 1,5 ml Methanol / 1,5 ml Tetrahydrofuran wird mit einer Lösung von 4,1 mg (0,041 mmol) Kaliumhydrogencarbonat in 1 ml Wasser versetzt. Es wird eingeengt und die wäßrige Lösung lyophilisiert.
Ausbeute: 25 mg, 100% der Theorie
MS (FAB): 610 (M⁺), 648 (M-1+K)

### Beispiel 6

### 6-Butoxy-2-{1-[2'-(1H-tetrazol-5-yl)-biphenyl-4-yl]-pentyl}-pyridin-3,5-dicarbonsäurediethylester

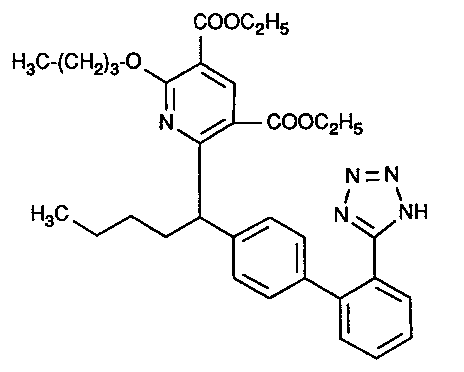

Analog Beispiel 1 und Beispiel 3 wird aus der Verbindung des Beispiels VI die Titelverbindung erhalten.
R_{f} (Kieselgel, Dichlormethan/Methanol 20:1) = 0,33

### Beispiel 7

### 1-Butyl-2-oxo-6-[2'-(1H-tetrazol-5-yl)-biphenyl-4-ylmethyl]-1,2-dihydropyridin-3,5-dicarbonsäurediethylester

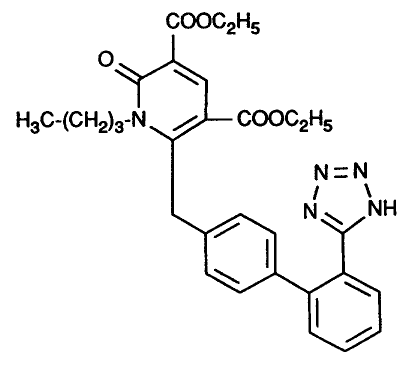

Analog Beispiel 3 wird die Titelverbindung ausgehend von der Verbindung des Beispiels 2 erhalten.
MS (FAB): 530, 531

### Beispiel 8

### 1-Butyl-2-oxo-6-[2'-(1H-tetrazol-5-yl)-biphenyl-4-ylmethyl]-1,2-dihydropyridin-3,5-dicarbonsäurediethylester-Kaliumsalz

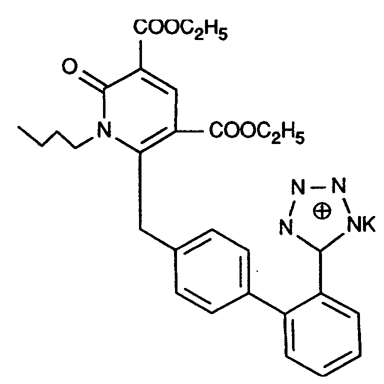

Analog Beispiel 5 wird aus der Verbindung des Beispiels 7 die Titelverbindung erhalten.
MS (FAB): 530 (M+1), 568 (M+K), 606 (M-1+2K), 646 (M+3K)

### Beispiel 9

### 1-Butyl-2-oxo-6-[2'-(1H-tetrazol-5-yl)-biphenyl-4-ylmethyl]-1,2-dihydropyridin-3,5-dicarbonsäure

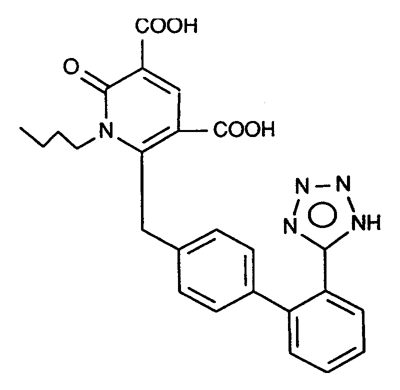

Eine Lösung von 100 mg (0,19 mmol) der Verbindung aus Beispiel 7 in Ethanol wird mit 200 ml (3,5 mmol) Kaliumhydroxid bei 20°C über Nacht gerührt. Es wird zur Hälfte eingeengt und mit konz. Salzsäure versetzt, wobei ein farbloser Feststoff ausfällt. Nach Absaugen und Trocknen im Hochvakuum werden 80 mg der Titelverbindung erhalten.

### Beispiel 10

### 1-Butyl-2-oxo-6-[2'-(1H-tetrazol-5-yl)-biphenyl-4-ylmethyl]-1,2-dihydropyridin-3,5-dicarbonsäure dikaliumsalz

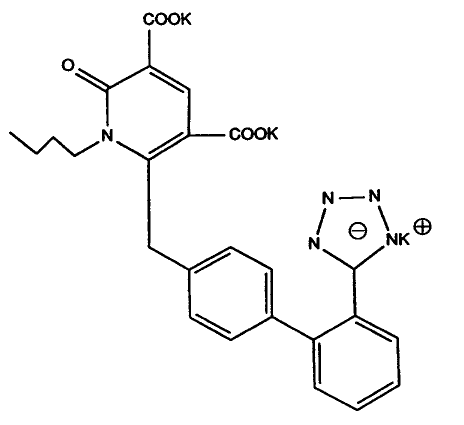

Eine Lösung von 50 mg (0,1 mmol) der Verbindung aus Beispiel 9 und 3,18 ml (0,32 mmol) einer 0,1 N Kaliumhydroxidlösung in Wasser wird gefriergetrocknet und über Phosphorpentoxid im Hochvakuum getrocknet.
Ausbeute: 63 mg (100% der Theorie)
MS (FAB): 474 (M+1), 512 (M+K), 550 (M+2K-H), 588 (M+3K)

### Beispiel 11

### 1-Butyl-2-oxo-6-[2'-(2-triphenylmethyl-tetrazol-5-yl)-biphenyl-4-yl-methyl]-1,2-dihydropyridin-3,5-dicarbonsäure-3-ethyl-5-methylester

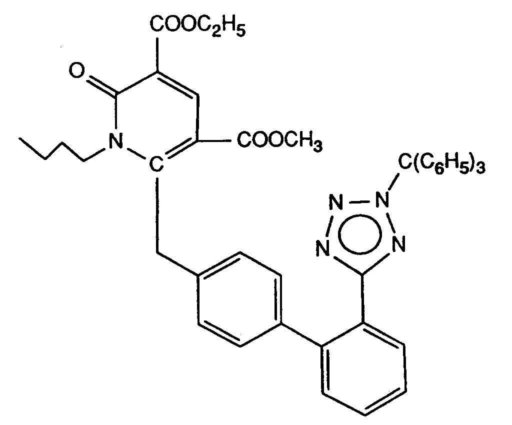

Analog Beispiel 1 wird die Titelverbindung ausgehend von der Verbindung des Beispiels XII erhalten.
Ausbeute: 21,5 %
R_{f} (Petrolether/Essigester 2:1, Kieselgel) = 0,37

### Beispiel 12

### 1-Butyl-2-oxo-6-[2'-(1H-tetrazol-5-yl)-biphenyl-4-ylmethyl]-1,2-dihydropyridin-3,5-dicarbonsäure-3-ethyl-5-methylester

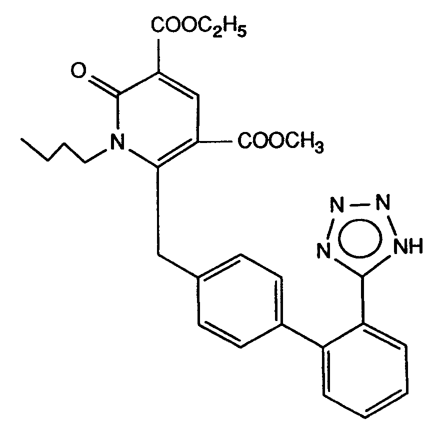

Analog Beispiel 3 wird die Titelverbindung ausgehend von der Verbindung des Beispiels 11 erhalten.
Ausbeute: 43 % der Theorie
MS (FAB): 516 (M+1)

## Patentansprüche

1. Substituierte Pyridine und 2-Oxo-1,2-dihydropyridine der allgemeinen Formel 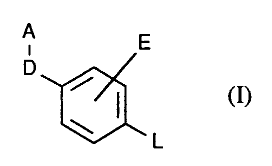 in welcher
A für einen Rest der Formel 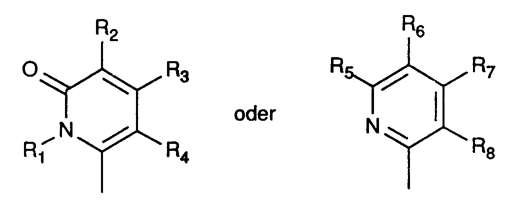 steht, worin
R¹ geradkettiges oder verzweigtes Alkyl mit bis zu 10 Kohlenstoffatomen bedeutet, das gegebenenfalls durch Cycloalkyl mit 3 bis 6 Kohlenstoffatomen, Hydroxy oder durch geradkettiges oder verzweigtes Alkoxy oder Alkylthio mit jeweils bis zu 6 Kohlenstoffatomen substituiert ist, oder
Cycloalkyl mit 3 bis 6 Kohlenstoffatomen bedeutet,
R², R⁵ und R⁶ gleich oder verschieden sind und
Wasserstoff, Hydroxy, Nitro, Cyano, Formyl oder Halogen bedeuten, oder
geradkettiges oder verzweigtes Alkyl, Alkenyl, Alkinyl, Alkoxy oder Alkylthio mit jeweils bis zu 8 Kohlenstoffatomen bedeuten, die gegebenenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5-bis 7-gliedrigen, gesättigten oder ungesättigten Heterocyclus mit bis zu 3 Heteroatomen aus der Reihe S, N oder O substituiert sind, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können, oder
geradkettiges oder verzweigtes Acyl oder Alkoxycarbonyl mit jeweils bis zu 8 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 6 Kohlenstoffatomen substituiert ist, das seinerseits durch Cyano, Halogen, Carboxy, Phenoxycarbonyl, Hydroxy oder durch geradkettiges oder verzweigtes Alkoxy oder Alkoxycarbonyl mit jeweils bis zu 6 Kohlenstoffatomen substituiert sein kann, oder
eine Gruppe der Formel 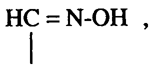 -NR⁹R¹⁰, -CO-NR¹¹R¹², -CH₂-OR¹³ oder -S(O)ₐ-R¹⁴ bedeuten
worin
R^{9,} R¹⁰, R¹¹ und R¹² gleich oder verschieden sind und Wasserstoff, Cycloalkyl mit 3 bis 8 Kohlenstoffatomen oder geradkettiges oder verzweigtes Alkyl mit bis zu 8 Kohlenstoffatomen bedeuten, das gegebenenfalls durch Phenyl substituiert ist,
oder
R⁹ und R¹⁰ gemeinsam mit dem Stickstoffatom einen 5- bis 7-gliedrigen, gesättigten Heterocyclus mit bis zu 2 weiteren Heteroatomen aus der Reihe S, N oder O bilden,
R¹³ geradkettiges oder verzweigtes Acyl mit bis zu 6 Kohlenstoffatomen oder Benzoyl bedeutet,
R¹⁴ geradkettiges oder verzweigtes Alkyl mit bis zu 8 Kohlenstoffatomen bedeutet,
a eine Zahl 1 oder 2 bedeutet,
R³ und R⁷ gleich oder verschieden sind und
Wasserstoff, Hydroxy, Carboxy, geradkettiges oder verzweigtes Alkoxy oder Alkoxycarbonyl mit jeweils bis zu 8 Kohlenstoffatomen bedeuten, oder
eine Gruppe der Formel -NR¹⁵R¹⁶ bedeuten,
worin
R¹⁵ und R¹⁶ die oben angegebene Bedeutung von R⁹ und R¹⁰ haben und mit dieser gleich oder verschieden sind,
oder
Aryl mit 6 bis 10 Kohlenstoffatomen bedeuten, das gegebenenfalls bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert ist, oder
geradkettiges oder verzweigtes Alkyl oder Alkenyl mit jeweils bis zu 8 Kohlenstoffatomen bedeuten, die gegebenenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können,
R⁴ Wasserstoff, Nitro, Carboxy oder geradkettiges oder verzweigtes Alkoxycarbonyl mit bis zu 8 Kohlenstoffatomen bedeutet, oder eine Gruppe der Formel -NR¹⁷R¹⁸ bedeutet,
worin
R¹⁷ und R¹⁸ die oben angegebene Bedeutung von R⁹ und R¹⁰ haben und mit diesen gleich oder verschieden sind,
oder
geradkettiges oder verzweigtes Alkyl oder Alkenyl mit jeweils bis zu 8 Kohlenstoffatomen bedeutet, die gegebenfalls bis zu 3-fach gleich oder verschieden durch Hydroxy, Cyano, Halogen, Carboxy, geradkettiges oder verzweigtes Alkoxy, Acyl oder Alkoxycarbonyl mit jeweils bis zu 6 Kohlenstoffatomen, oder durch Cycloalkyl mit 3 bis 6 Kohlenstoffatomen, Benzyl, Phenyl, Phenoxy, Benzoyl oder durch einen 5- bis 7-gliedrigen, gesättigten oder ungesättigten Heterocyclus mit bis zu 3 Heteroatomen aus der Reihe S, N oder O substituiert ist, wobei die Cyclen ihrerseits bis zu 2-fach gleich oder verschieden durch Trifluormethyl, Trifluormethoxy, Halogen, Nitro, Cyano, Hydroxy, Hydroxymethyl oder durch geradkettiges oder verzweigtes Alkyl oder Alkoxy mit jeweils bis zu 6 Kohlenstoffatomen substituiert sein können,
R⁸ die oben angegebene Bedeutung von R¹ und R⁴ hat und mit diesen gleich oder verschieden ist,
D für die 〉C=O -Gruppe steht, oder
für eine Gruppe der Formel 〉CH-T steht,
worin
T Wasserstoff oder geradkettiges oder verzweigtes Alkyl mit bis zu 8 Kohlenstoffatomen bedeutet,
E für Wasserstoff, Halogen, Cyano, Nitro, Trifluormethyl, Hydroxy, Trifluormethoxy, Amido oder für geradkettiges oder verzweigtes Alkyl, Alkoxy oder Alkoxycarbonyl mit jeweils bis zu 6 Kohlenstoffatomen steht,
L für einen Rest der Formel 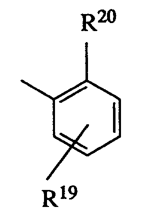 steht,
worin
R¹⁹ die oben angegebene Bedeutung von E hat und mit dieser gleich oder verschieden ist,
und
R²⁰ eine Gruppe der Formel -CO-R²¹, -SO₂R²², - CO-NR²³R²⁴, -NH-SO₂R²⁵ oder -SO₂-NR²⁶R²⁷ bedeutet,
worin
R²¹ Hydroxy oder geradkettiges oder verzweigtes Alkoxy mit bis zu 6 Kohlenstoffatomen bedeutet,
R²² Hydroxy, Trifluormethyl, geradkettiges oder verzweigtes Alkoxy oder Alkyl mit jeweils bis zu 6 Kohlenstoffatomen, Phenyl oder Benzyl bedeutet, das gegebenenfalls bis zu 2-fach gleich oder verschieden durch Halogen, Trifluormethyl oder geradkettiges oder verzweigtes Alkyl mit bis zu 4 Kohlenstoffatomen substituiert sein kann,
R²³ und R²⁴ gleich oder verschieden sind und die oben angegebene Bedeutung von R⁹ und R¹⁰ haben,
oder
R²³ Wasserstoff bedeutet
und
R²⁴ die Gruppe -SO₂R²² bedeutet,
worin
R²² die oben angegebene Bedeutung hat,
R²⁵ die oben angegebene Bedeutung von R²² hat und mit dieser gleich oder verschieden ist,
R²⁶ und R²⁷ die oben angegebene Bedeutung von R⁹ und R¹⁰ haben und mit diesen gleich oder verschieden sind,
oder
R²⁶ geradkettiges oder verzweigtes Alkyl mit bis zu 4 Kohlenstoffatomen oder Wasserstoff bedeutet
und
R²⁷ die oben angegebene Bedeutung von R²² hat und mit dieser gleich oder verschieden ist
oder
R²⁰ einen Rest der Formel 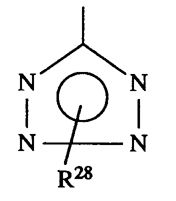 bedeutet,
worin
R²⁸ Wasserstoff oder geradkettiges oder verzweigtes Alkyl mit bis zu 8 Kohlenstoffatomen bedeutet, das gegebenenfalls durch geradkettiges oder verzweigtes Acyl mit bis zu 6 Kohlenstoffatomen substituiert ist oder die Triphenylmethylgruppe bedeutet,
und deren Salze.

2. Substituierte Pyridine und 2-oxo-1,2-dihydropyridine nach Anspruch 1, wobei
A für einen Rest der Formel 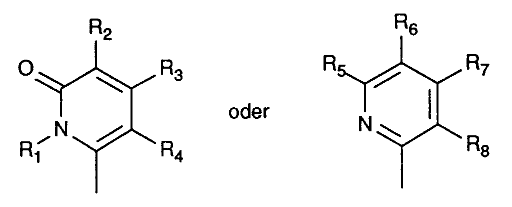 steht,
worin
R¹ Wasserstoff, geradkettiges oder verzweigtes Alkyl mit jeweils bis zu 8 Kohlenstoffatomen bedeutet, das gegebenenfalls durch Cyclopropyl, Cyclopentyl, Cyclohexyl, Hydroxy oder durch geradkettiges oder verzweigtes Alkoxy oder Alkylthio mit jeweils bis zu 4 Kohlenstoffatomen substituiert ist, oder
Cyclopropyl, Cyclopentyl oder Cyclohexyl bedeutet,
R², R⁵ und R⁶ gleich oder verschieden sind und
Wasserstoff, Hydroxy, Nitro, Cyano, Formyl, Fluor, Chlor, Brom oder Iod bedeuten, oder
geradkettiges oder verzweigtes Alkyl, Alkenyl, Alkinyl, Alkoxy oder Alkylthio mit jeweils bis zu 6 Kohlenstoffatomen bedeuten, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können, oder
geradkettiges oder verzweigtes Acyl oder Alkoxycarbonyl mit jeweils bis zu 6 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 5 Kohlenstoffatomen substituiert ist, das seinerseits durch Cyano, Fluor, Chlor, Brom, Carboxy, Hydroxy oder durch geradkettiges oder verzweigtes Alkoxy oder Alkoxycarbonyl mit jeweils bis zu 4 Kohlenstoffatomen substituiert sein kann, oder
eine Gruppe der Formel 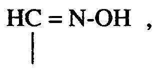 -NR⁹R¹⁰, -CO-NR¹¹R¹², -CH₂-OR¹³ oder -S(O)ₐ-R¹⁴ bedeuten
worin
R⁹, R¹⁰, R¹¹ und R¹² gleich oder verschieden sind und Wasserstoff, Cyclopropyl, Cyclopentyl, Cyclohexyl oder geradkettiges oder verzweigtes Alkyl mit bis zu 6 Kohlenstoffatomen bedeuten, das gegebenenfalls durch Phenyl substituiert ist,
oder
R⁹ und R¹⁰ gemeinsam mit dem Stickstoffatom einen Morpholinring bilden,
R¹³ geradkettiges oder verzweigtes Acyl mit bis zu 6 Kohlenstoffatomen oder Benzoyl bedeutet,
R¹⁴ geradkettiges oder verzweigtes Alkyl mit bis zu 6 Kohlenstoffatomen bedeutet,
a eine Zahl 1 oder 2 bedeutet,
R³ und R⁷ gleich oder verschieden sind und Wasserstoff, Hydroxy, Carboxy, geradkettiges oder verzweigtes Alkoxy oder Alkoxycarbonyl mit jeweils bis zu 6 Kohlenstoffatomen bedeutem, oder
eine Gruppe der Formel -NR¹⁵R¹⁶ bedeuten,
worin
R¹⁵ und R¹⁶ die oben angegebene Bedeutung von R⁹ und R¹⁰ haben und mit diesen gleich oder verschieden sind,
oder
Phenyl bedeuten, das gegebenenfalls durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein kann, oder
geradkettiges oder verzweigtes Alkyl oder Alkenyl mit jeweils bis zu 6 Kohlenstoffatomen bedeuten, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können,
R⁴ Wasserstoff, Nitro, Carboxy oder geradkettiges oder verzweigtes Alkoxycarbonyl mit bis zu 6 Kohlenstoffatomen bedeutet, oder
eine Gruppe der Formel -NR¹⁷R¹⁸ bedeutet,
worin
R¹⁷ und R¹⁸ die oben angegebene Bedeutung von R⁹ und R¹⁰ haben und mit diesen gleich oder verschieden sind,
oder
geradkettiges oder verzweigtes Alkyl oder Alkenyl mit jeweils bis zu 6 Kohlenstoffatomen bedeutet, die gegebenenfalls durch Hydroxy, Cyano, Fluor, Chlor, Brom, Carboxy, geradkettiges oder verzweigtes Alkoxy, Acyl oder Alkoxycarbonyl mit jeweils bis zu 4 Kohlenstoffatomen, oder durch Benzyl, Phenyl, Phenoxy, Benzoyl oder Thienyl substituiert sind, wobei die Cyclen ihrerseits durch Trifluormethoxy, Trifluormethyl, Hydroxymethyl, Fluor, Chlor, Brom, Iod oder durch geradkettiges oder verzweigtes Alkyl oder Alkoxy mit jeweils bis zu 6 Kohlenstoffatomen substituiert sein können,
R⁸ die oben angegebene Bedeutung von R¹ und R⁴ hat und mit diesen gleich oder verschieden ist,
D für die 〉C=O -Gruppe steht, oder
für eine Gruppe der Formel 〉CH-T steht,
worin
T Wasserstoff oder geradkettiges oder verzweigtes Alkyl mit bis zu 7 Kohlenstoffatomen bedeutet,
E für Wasserstoff, Fluor, Chlor, Brom, Cyano, Nitro, Methoxy, Amido, Trifluormethyl, Trifluormethoxy oder für geradkettiges oder verzweigtes Alkyl oder Alkoxycarbonyl mit jeweils bis zu 4 Kohlenstoffatomen steht,
L für einen Rest der Formel 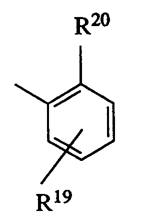 steht,
worin
R¹⁹ die oben angegebene Bedeutung von D hat und mit dieser gleich oder verschieden ist
und
R²⁰ eine Gruppe der Formel -CO-R²¹, -SO₂R²², - CO-NR²³R²⁴, -NH-SO₂R²⁵ oder -SO₂-NR²⁶R²⁷ bedeutet,
worin
R²¹ Hydroxy oder geradkettiges oder verzweigtes Alkoxy mit bis zu 4 Kohlenstoffatomen bedeutet,
R²² geradkettiges oder verzweigtes Alkyl mit bis zu 4 Kohlenstoffatomen, Benzyl, Trifluormethyl oder p-Tolyl bedeutet,
R²³ und R²⁴ gleich oder verschieden sind und die oben angegebene Bedeutung von R⁹ und R¹⁰ haben oder
R²³ Wasserstoff bedeutet und
R²⁴ die Gruppe -SO₂R²² bedeutet,
worin
R²² die oben angegebene Bedeutung hat,
R²⁵ die oben angegebene Bedeutung von R²² hat und mit dieser gleich oder verschieden ist,
R²⁶ und R²⁷ die oben angegebene Bedeutung von R⁹ und R¹⁰ haben und mit diesen gleich oder verschieden sind,
oder
R²⁶ geradkettiges oder verzweigtes Alkyl mit bis zu 4 Kohlenstoffatomen oder Wasserstoff bedeutet
und
R²⁷ die oben angegebene Bedeutung von R²² hat und mit dieser gleich oder verschieden ist,
oder
R²⁰ einen Rest der Formel 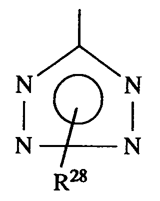 bedeutet,
worin
R²⁸ Wasserstoff oder geradkettiges oder verzweigtes Alkyl mit bis zu 6 Kohlenstoffatomen bedeutet, das gegebenenfalls durch geradkettiges oder verzweigtes Acyl mit bis zu 4 Kohlenstoffatomen substituiert ist oder die Triphenylmethylgruppe bedeutet,
und deren Salze.

3. Substituierte Pyridine und 2-oxo-1,2-dihydropyridine nach Anspruch 1
worin
A für einen Rest der Formel 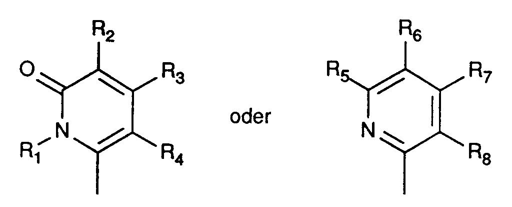 steht,
worin
R¹ Wasserstoff oder geradkettiges oder verzweigtes Alkyl mit bis zu 6 Kohlenstoffatomen bedeutet, das gegebenenfalls durch Hydroxy, geradkettiges oder verzweigtes Alkoxy mit jeweils bis zu 3 Kohlenstoffatomen substituiert ist oder
Cyclopropyl, Cyclopentyl oder Cyclohexyl bedeutet,
R² und R⁶ gleich oder verschieden sind und Wasserstoff, Cyano, Formyl, Fluor, Chlor, Brom oder Iod bedeuten, oder
für geradkettiges oder verzweigtes Alkyl oder Alkoxy mit jeweils bis zu 4 Kohlenstoffatomen stehen, oder
geradkettiges oder verzweigtes Alkoxycarbonyl mit jeweils bis zu 4 Kohlenstoffatomen, Phenoxycarbonyl, Benzyloxycarbonyl oder Carboxy bedeuten, oder
Tetrazolyl bedeuten, das gegebenenfalls durch Triphenylmethyl oder durch geradkettiges oder verzweigtes Alkyl mit bis zu 4 Kohlenstoffatomen substituiert ist, oder -NR⁹R¹⁰, -CO-NR¹¹R¹², -CH₂-OR¹³ bedeuten
worin
R⁹, R¹⁰, R¹¹ und R¹² gleich oder verschieden sind und Wasserstoff, Cyclopropyl, Cyclopentyl oder geradkettiges oder verzweigtes Alkyl mit bis zu 4 Kohlenstoffatomen bedeuten, das gegebenenfalls durch Phenyl substituiert ist,
R¹³ geradkettiges oder verzweigtes Acyl mit bis zu 4 Kohlenstoffatomen oder Benzoyl bedeutet,
R⁵ Hydroxy oder geradkettiges oder verzweigtes Alkyl oder Alkoxy mit jeweils bis zu 6 Kohlenstoffatomen bedeutet,
R³ und R⁷ gleich oder verschieden sind und Wasserstoff, Hydroxy, Carboxy, geradkettiges oder verzweigtes Alkoxy oder Alkoxycarbonyl mit jeweils bis zu 4 Kohlenstoffatomen bedeuten, oder
eine Gruppe der Formel -NR¹⁵R¹⁶ bedeuten,
worin
R¹⁵ und R¹⁶ die oben angegebene Bedeutung von R⁹ und R¹⁰ haben und mit diesen gleich oder verschieden sind,
oder
Phenyl, Vinyl oder geradkettiges oder verzweigtes Alkyl mit bis zu 5 Kohlenstoffatomen bedeuten,
R⁴ Wasserstoff, Carboxy oder geradkettiges oder verzweigtes Alkoxycarbonyl mit bis zu 4 Kohlenstoffatomen bedeutet, oder
eine Gruppe der Formel -NR¹⁷R¹⁸ bedeutet,
worin
R¹⁷ und R¹⁸ die oben angegebene Bedeutung von R⁹ und R¹⁰ haben und mit diesen gleich oder verschieden sind,
oder
Vinyl oder geradkettiges oder verzweigtes Alkyl mit bis zu 4 Kohlenstoffatomen bedeutet,
R⁸ die oben angegebene Bedeutung von R¹ und R⁴ hat und mit diesen gleich oder verschieden ist,
D für die 〉C=O-Gruppe steht, oder
für eine Gruppe der Formel 〉CH-T steht,
worin
T Wasserstoff oder geradkettiges oder verzweigtes Alkyl mit bis zu 5 Kohlenstoffatomen bedeutet,
E für Wasserstoff, Fluor, Chlor, Brom, Cyano, Methoxy, Trifluormethyl oder geradkettiges oder verzweigtes Alkyl oder Alkoxycarbonyl mit jeweils bis zu 3 Kohlenstoffatomen steht,
und
L für einen Rest der Formel 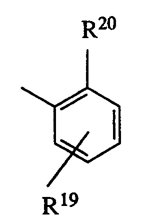 steht,
worin
R¹⁹ Wasserstoff bedeutet,
und
R²⁰ eine Gruppe der Formel -CO-R²¹ bedeutet,
worin
R²¹ Hydroxy oder geradkettiges oder verzweigtes Alkoxy mit bis zu 4 Kohlenstoffatomen bedeutet,
oder
R²⁰ den Tetrazolylrest der Formel 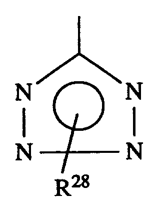 bedeutet,
worin
R²⁸ Wasserstoff oder geradkettiges oder verzweigtes Alkyl mit bis zu 4 Kohlenstoffatomen bedeutet,
oder die Triphenylmethylgruppe bedeutet,
und deren Salze.

4. Substituierte Pyridine und 2-oxo-1,2-dihydropyridine nach Anspruch 1
worin
A für einen Rest der Formel 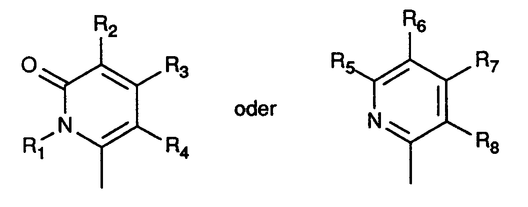 steht,
worin
R¹ geradkettiges oder verzweigtes Alkyl mit bis zu 4 Kohlenstoffatomen bedeutet,
R² und R⁶ gleich oder verschieden sind und Wasserstoff, Carboxy oder Alkoxycarbonyl mit bis zu 4 Kohlenstoffatomen bedeuten,
R⁵ Hydroxy oder geradkettiges oder verzweigtes Alkyl oder Alkoxy mit bis zu 4 Kohlenstoffatomen bedeutet,
R³ und R⁷ gleich oder verschieden sind und Wasserstoff, Hydroxy, Carboxy oder geradkettiges oder verzweigtes Alkoxy oder Alkoxycarbonyl mit jeweils bis zu 4 Kohlenstoffatomen bedeuten,
R⁴ Wasserstoff, Carboxy oder geradkettiges oder verzweigtes Alkoxycarbonyl mit bis zu 4 Kohlenstoffatomen bedeutet,
R⁸ die oben angegebene Bedeutung von R¹ und R⁴ hat und mit diesen gleich oder verschieden ist,
D für die 〉C=O-Gruppe steht, oder
für eine Gruppe der Formel 〉CH-T steht,
worin
T Wasserstoff oder geradkettiges oder verzweigtes Alkyl mit bis zu 4 Kohlenstoffatomen bedeutet,
E für Wasserstoff, Fluor, Chlor, Trifluormethyl oder Cyano steht,
und
L für einen Rest der Formel 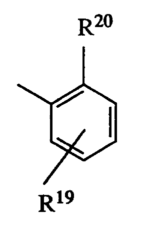 steht,
worin
R¹⁹ Wasserstoff bedeutet,
und
R²⁰ eine Gruppe der Formel -CO-R²¹, bedeutet,
worin
R²¹ Hydroxy oder geradkettiges oder verzweigtes Alkoxy mit bis zu 4 Kohlenstoffatomen bedeutet,
oder
R²⁰ den Tetrazolylrest der Formel 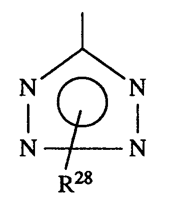 bedeutet,
worin
R²⁸ Wasserstoff oder die Triphenylmethylgruppe bedeutet,
und deren Salze.

5. Substituierte Pyridine und 2-oxo-1,2-dihydropyridine nach Anspruch 1 bis 4 zur Anwendung als Arzneimittel.

6. Verfahren zur Herstellung von substituierten Pyridinen und 2-oxo-1,2-dihydropyridinen nach Anspruch 1 bis 4,
dadurch gekennzeichnet, daß man
Verbindungen der allgemeinen Formel (II) 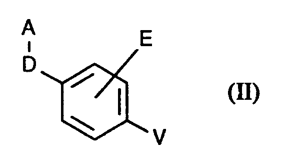 in welcher
A, D und E die oben angegebene Bedeutung haben
und
V für eine typische Abgangsgruppe, wie beispielsweise Brom, Jod, Methan-, Toluol-, Fluor- oder Trifluormethansulfonyloxy, vorzugsweise für Brom steht,
mit Verbindungen der allgemeinen Formel (III) oder (IIIa) 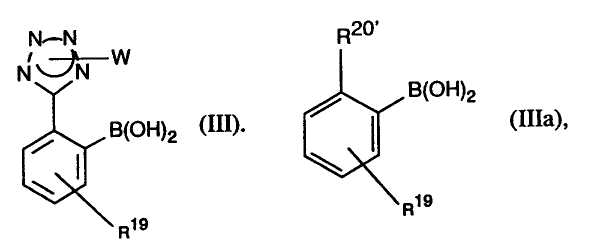 in welcher
R¹⁹ die oben angegebene Bedeutung hat,
W für Wasserstoff oder für die Triphenylmethylgruppe steht,
und
R^{20'} die oben angegebene Bedeutung von R²⁰ hat, aber nicht für den Tetrazolylrest steht,
in inerten Lösemitteln, in Anwesenheit einer Base und metallkatalysiert umsetzt,
und anschließend im Fall daß W für die Triphenylmethylgruppe steht mit Säuren in organischen Lösemitteln und/oder Wasser nach üblichen Bedingungen abspaltet,
und gegebenenfalls im Fall der unter dem Substituenten R²⁰ aufgeführten Reste nach Verseifung der jeweiligen Ester, beispielsweise durch Amidierung oder Sulfoamidierung, nach üblichen Methoden derivatisiert,
und im Fall der Salze bevorzugt ausgehend vom freien Tetrazol (R²⁸/W = H) mit Säuren oder Basen umsetzt,
und im Fall der freien Säure R²⁰ = CO₂H und des freien Tetrazols R²⁸ = H, ausgehend von den Salzen mit Säuren umsetzt,
und gegebenenfalls auch die übrigen Substituenten nach bekannten Methoden auf jeder Verfahrensstufe variiert.

7. Arzneimittel enthaltend mindestens ein substituiertes Pyridin oder 2-oxo-1,2-dihydropyridin nach Anspruch 1 bis 4 sowie geeignete Formulierungshilfsstoffe.

8. Arzneimittel nach Anspruch 7 zur Behandlung der arteriellen Hypertonie und Atherosklerose.

9. Verwendung von substituierten Pyridinen und 2-oxo-1,2-dihydropyridinen nach Anspruch 1 bis 4 zur Herstellung von Arzneimitteln.

10. Verwendung nach Anspruch 9 zur Herstellung von Arzneimitteln zur Behandlung der arteriellen Hypertonie und Atherosklerose.
